(19) Europäisches Patentamt
European Patent Office
Office européen des brevets

(11) Publication number: **0 110 405**
**B1**

(12) **EUROPEAN PATENT SPECIFICATION**

(45) Date of publication of patent specification: **27.12.90**

(21) Application number: **83112031.6**

(22) Date of filing: **30.11.83**

(51) Int. Cl.[5]: **C 07 C 69/76**, C 07 D 213/30, C 07 D 215/14, C 07 C 47/575, C 07 C 49/84, C 07 D 213/79, C 07 C 43/23, C 07 C 65/21, C 07 C 67/31

(54) **Antiinflammatory/antiallergic compounds.**

(30) Priority: **01.12.82 US 445876**

(43) Date of publication of application:
**13.06.84 Bulletin 84/24**

(45) Publication of the grant of the patent:
**27.12.90 Bulletin 90/52**

(84) Designated Contracting States:
**AT BE CH DE FR GB IT LI LU NL SE**

(56) References cited:
**EP-A-0 113 587**
**FR-A-2 253 511**
**US-A-2 862 956**
**US-A-4 360 700**

**JOURNAL OF PHARMACEUTICAL SCIENCES, vol. 62, no. 6, June 1973, pages 952-957; M.R. BOOTS et al.: "Hypocholesterolemic agents II: Inhibition of beta-hydroxy-beta-methylglutaryl coenzyme A reductase by arylalkyl hydrogen succinates and glutarates"**

**The file contains technical information submitted after the application was filed and not included in this specification**

(73) Proprietor: **RORER INTERNATIONAL (OVERSEAS) INC. (a Delaware corporation)
P.O. Box 145
Lewes Delaware 19958 (US)**

(72) Inventor: **Musser, John H
15 Steiner Drive
Mahopac New York (US)**
Inventor: **Chakraborty, Utpal Ranjan
51 Minuteman Circle
Orangeburg New York (US)**

(74) Representative: **Patentanwälte Grünecker, Kinkeldey, Stockmair & Partner
Maximilianstrasse 58
D-8000 München 22 (DE)**

**EP 0 110 405 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to lipoxygenase inhibitor compounds possessing anti-inflammatory and anti-allergic activities and having the general formula I

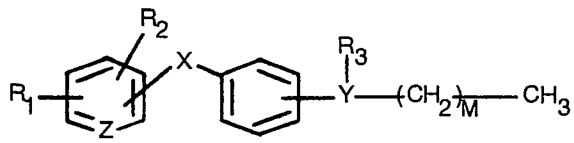

(I)

and pharmaceutically acceptable salts thereof, wherein

$R_1$ and $R_2$ are independently hydrogen, alkyl, carboxy, carboxyalkyl, carbalkoxyalkyl, carbalkoxy, formyl, amino, aminoalkyl, alkylamino, carboxamide, halo, trihalomethyl, hydroxy, or $R_1$ and $R_2$ when taken together with the carbon atoms to which they are attached form a phenyl ring;

Z is CH or N;

X is —O—, —$CH_2Z_1$—, —HC=CH—, —C≡C— or —$CH_2Z_1CH_2$;

Y is —$CHCH_2$—, —C=CH—, —$CHCHR_5$ or —$CHC(R_5)_2$— when $R_3$ is monovalent or —C—$CH_2$ when $R_3$ is O;

$R_3$ is O, OH, $OR_4$, NH, $HNR_4$ or $N(R_4)_2$; and

M is an integer from 0 to 5;

wherein $Z_1$ is O, S or $NR_4$; $R_4$ is H, alkyl, pyranyl or aryl; and $R_5$ is H, alkyl or fluorine, with the proviso that when $R_3$ is OH, M is 3 and the relationship of X and Y is meta or para then one of $R_1$ or $R_2$ is other than hydrogen.

M. R. Boots et al: Hypocholesterolemic Agents II, Journal of Pharmaceutical Sciences, Volume 62 (1973), pages 952—957, discloses a series of arylalkyl hydrogen succinates and glutarates and a method for the preparation of said substances.

US—A—4360700 discloses a process for making 1-(3-benzyloxyphenyl)-1-1-dimethylheptane, a valuable intermediate for the preparation of analgesic agents.

US—A—2862956 discloses a process of producing 2-hydroxy methyl benzoic acid compounds.

FR—A—2253511 discloses specific ketones, their preparation and their use as therapeutic agents.

In the general formula I the alkyl groups in alkyl carboxy, carboxyalkyl, alkyl carboxyalkyl, alkylamino, aminoalkyl, carbalkoxy, alkyl carbalkoxy, and in $NR_4$ and $CR_5$ contain from 1 to 6 carbon atoms and have either the straight or branched structure. Such groups include methyl, ethyl, propyl, isopropyl, butyl, isobutyl, amyl, isoamyl, hexyl, vinyl, alkyl, propenyl and ethynyl.

The halo groups in halo and trihalomethyl contain F, Cl, Br or I.

The aryl groups in aryl are phenyl or naphthyl.

The preferred compounds are those in which: $R_1$ and $R_2$ are independently alkylcarboxy or $R_1$ and $R_2$ form a phenyl ring; the alkyl group in alkylcarboxy is methyl; Z is CH or N; X is $CH_2Z_1$; $Z_1$ is O; Y is $CHCH_2$; $R_3$ is OH or $HNR_4$ and M is an integer from 2 to 3 inclusive.

The compounds of the present invention can be prepared by various synthetic methods as exemplified by the following:

1. Condensation of a compound of formula II

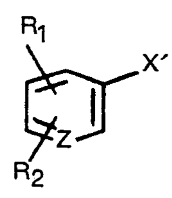

(II)

with a compound of formula III

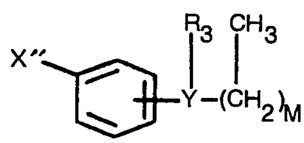

(III)

wherein X′ and X″ are —$CH_2Hal$, Hal, OH, $CH_2OH$, SH or $CH_2SH$, through elimination of X′ and X″ with a residue X remaining after condensation. Such condensation reactions are known and occur between, for example, —$CH_2Hal$ and —OH with the elimination of hydrogen halide and formation of the bridging group —$CH_2O$— between the two rings of the resulting product. Substituting simply halogen in place of

$—CH_2Hal—$ results in the diphenyl ether structure (where X is —O—). This reaction is known as Ullman ether synthesis. Substituting the analogous sulfuric intermediates provides the sulfur analogs of the aforesaid compounds (where X is —S— or $—CH_2S—$).

A variation of this synthetic route employs the Grignard reagent in place of the halogen-containing substituent.

2. When X is unsaturated hydrocarbyl

(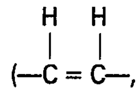, or —C≡C—)

a compound of formula II in which X′ is vinyl or acetylenyl is condensed with a compound of formula III in which X″ is halogen, usually in the presence of an active metal (Na, Li, etc.). Alternatively, X′ and X″ of course can be reversed, i.e., X′ can be halogen and X″ the unsaturated hydrocarbyl group.

3. When X is —CH=N—, X′ and X″ can be —CHO and $NH_2$ and anil formation will result in production of the final product.

4. When X is an amide or ester linkage, i.e., —COZ—, X′ and X″, of course, are COOH, and $Z_1H$, on either formula II or formula III, by classical ester or amide formation methods.

5. When X is a carbonyl, i.e., C=O, X′ and X″ are H or an acylating group on either formula II or formula III by classical ketone formation reaction, e.g., by Friedl-Crafts reaction; or alternatively by oxidation of the compound in which X is —CHOH—.

6. When X includes $—NR_4—$ by classical methods of forming diphenyl amines which include reaction between the compound (either of formula II or III) in which X′ or X″ is $—NR_4H—$ with the compound (formula II or III) in which X′ or X″ is halogen.

7. Alternatively, substituent

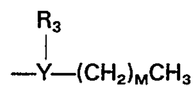

can be added to compounds of formula IV

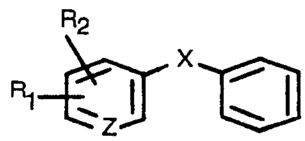

(IV)

using classical acylation or alkylation condensation reactions, for example, an acylating derivative of an acid

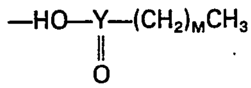

with a Friedl-Crafts catalyst to provide compounds in which

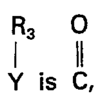

from which compounds in which $R_3$ is other than O can be prepared by known reactions, e.g., reduction of the carbonyl oxygen to —OH using lithium aluminum hydride and similar reducing agents.

Substituents $R_1$ and $R_2$ can be present in the starting intermediates in the foregoing synthetic routes or alternatively can be added by suitable substitution reactions using the starting compounds of formula I herein in which $R_1$ and/or $R_2$ are hydrogen.

The described reactions are performed using classical organic reactions known to those skilled in the art. Of course, reactive groups where present are preferably blocked by known methods to avoid competing or secondary reactions.

The aforesaid reactions are carried out in an organic solvent for the reactive starting materials at temperatures ranging from room temperature up to the reflux temperature of the reaction mixture. In condensation reactions in which hydrogen halide is formed, a hydrogen halide acceptor, i.e., usually a basic compound such as an organic amine, is present to facilitate the reaction. In those reactions where a Friedl-Crafts catalyst is employed, aluminum chloride or zinc-chloride are generally useful.

The final products are recovered from the reaction mixtures and subsequently purified by art-

recognized procedures, e.g., column chromatographic techniques.

The invention will be more fully illustrated in the examples that follow.

Example 1A

Methyl-2-Bromomethylbenzoate

A water jacketted, immersion photolysis vessel equipped with a $N_2$ inlet, dropping funnel and a reflux condenser was charged with 1 l of carbon tetrachloride and 127 g (0.847 mol) of 2-methyl toluate (Pfaltz and Bauer M29200). A solution of 400 ml carbon tetrachloride and 43.5 ml (0.849 mol) of bromine was added to the dropping funnel. After the solution had been heated to reflux the bromine solution was slowly added while the solution was irradiated with a 600 W incandescent lamp. After the addition of the bromine solution was complete, the lamp was turned off and the solution cooled. The carbon tetrachloride was removed under reduced pressure. The resultant oil was crystallized from 250 ml of a (1:1) solution of diethyl ether and hexane. The solid was collected and washed with hexane to yield 119 g (61%) of product.

In like manner as above using appropriate starting materials, the following compounds were prepared:

Methyl-3-bromomethylbenzoate;

Methyl-4-bromomethylbenzoate; and

Methyl-3-bromomethylphenylacetate.

Example 1B

1-(3-Hydroxyphenyl)-1-pentanol

A dried 1 l 3-neck flask equipped with a $N_2$ inlet, reflux condenser, mechanical stirrer and a 500 ml dropping funnel was charged with 24.3 g (1.0 mol) of magnesium and 50 ml of anhydrous ether. To this was added 15 g (0.11 mol) of 1-bromobutane (Aldrich 23,988-7) and one crystal of iodine. The dropping funnel was charged with 122 g (0.89 mol) of 1-bromobutane and 100 ml of anhydrous ether. After the contents of the reaction flask began to reflux, the flask was cooled with a water/ice bath and the 1-bromobutane solution was added at such a rate as to maintain a gentle reflux. After the addition was complete the reaction mixture was refluxed for one-half hour, then cooled to 0°C in an ice/water bath. The dropping funnel was then charged with 38.0 g (0.311 mol) of 3-hydroxybenzaldehyde (Aldrich H 1,980-8) and 250 ml of anhydrous ether. This slurry was added over a 1 h period. After the addition the reaction mixture was allowed to warm up to room temperature and was left overnight. The reaction mixture was neutralized with 900 ml of 5% aqueous HCl. The reaction mixture was extracted with 2 × 500 ml of ethyl acetate, the organic extracts combined, washed with 1 l of water, dried over anhydrous sodium sulfate and concentrated under reduced pressure. The crude product was recrystallized from ethyl acetate to yield 44.0 g (79%) of 1-(3-hydroxyphenyl)-1-pentanol, m.p. 120—122°C.

In like manner as above, using appropriate starting materials and reagents, the following compounds were prepared:

1-(3-Hydroxyphenyl)-1-hexanol;

1-(2-hydroxyphenyl)-1-hexanol;

2-(3-hydroxyphenyl)-2-heptene;

1-(4-hydroxyphenyl)-1-hexene; and

phenyl-3-[1-(hydroxy)hexyl]benzyl ether.

Example 1C

Methyl-2-[[3-(1-hydroxypentyl)phenoxy]methyl]benzoate

To a 1 l 3-neck round bottom flask was added 45.2 g (0.197 mol) of methyl *O*-bromomethylbenzoate 35.5 g (0.197 mol) of 1-(3-hydroxyphenyl)-1-pentanol, 3.0 g (0.020 mol) of sodium iodide, 64.3 g (0.198 mol) of cesium carbonate and 500 ml of acetone. This slurry was refluxed for 3½ days. At which time the reaction mixture was cooled, solid removed by suction filtration and the solvent removed under reduced pressure. The resulting oil was partitioned between 10% aqueous HCl and ethyl acetate. The organic extract was washed with 200 ml of water, dried over anhydrous sodium sulfate and concentrated to yield 65.2 g of oil. A silica gel chromatography using hexane/chloroform (1:2) as eluent afforded 54.7 g (85%) of product as an oil.

In like manner as above, using appropriate starting materials and reagents, the following compounds were prepared:

Methyl-3-[[3-(1-hydroxyhexyl)phenoxy]methyl]benzoate;

Methyl-2-[[3-(1-hydroxyhexyl)phenoxy]methyl]benzoate;

Methyl-4-[[3-(1-hydroxyhexyl)phenoxy]methyl]benzoate;

Methyl-3-[[2-(1-hydroxyhexyl)phenoxy]methyl]benzoate;

Methyl-3-[[3-(1-hydroxyhexyl)phenoxy]methyl]phenylacetate;

3-[[3-(1-hydroxyhexyl)phenoxy]methyl]benzonitrile;

Methyl-2-[4-[[3-(1-hydroxyhexyl)phenoxy]methyl]phenoxy]acetate;

Benzyl-3-[1-hydroxyl)hexyl]phenyl ether;

Methyl-3-[[3-1-methyl-1-hexenyl)phenoxy]methyl benzoate;

Methyl-3-[[4-(1-hexenyl)phenoxy]methyl benzoate;

6-[[3-(1-hydroxyhexyl)phenoxy]methyl]picolinyl nitrile; and

Phenyl-3-cyanobenzyl ether.

Example 2

2-[[3-(1-Hydroxyhexyl)phenoxy]methyl]benzoic acid

A solution of methyl-2-[[3-(1-hydroxyhexyl]phenoxy]-methyl]benzoate (1.0 g) in 50 ml of methanol was treated with 1*N* aqueous sodium hydroxide. The reaction was stirred for 1 h at room temperature. The mixture was washed with ethyl ether, acidified with 5% aqueous hydrochloric acid and extracted with chloroform. The chloroform extract was dried ($MgSO_4$) and concentrated to a solid (0.7 g, 73% yield), m.p. 76—80°C.

In like manner as above, using appropriate starting material, the following compounds were prepared:

3-[[3-(1-hydroxyhexyl)phenoxy]methyl]benzoic acid;

2-[[3-(1-hydroxyhexyl)phenoxy]methyl]benzoic acid, m.p. 76—80;

2-[[3-(1-hydroxyhexyl)phenoxy]methyl]benzoic acid, m.p. 115—115.5;

4-[[3-(1-hydroxyhexyl)phenoxy]methyl]benzoic acid, m.p. 105—6;

3-[[3-(1-hydroxyhexyl)phenoxy]methyl]phenyl acetic acid; and

6-[[3-(1-hydroxyhexyl)phenoxy]methyl]picolinic acid, m.p. 111—113.

Example 3

Methyl-3-[[3-(1-acetoxyhexyl)phenoxy]methyl]benzoate

To a solution of methyl-3-[[3-(1-hydroxyhexyl)phenoxy]methyl]benzoate (1.7 g) in pyridine at 0°C was added acetic anhydride (2.7 ml). The reaction was stirred for 4 days at room temperature. The solvent was removed *in vacuo* and the remaining oil was purified by HPLC on silica gel using a hexanes/ethyl acetate in 9:1 ratio as an eluent (1.1 g, 57% yield).

Example 4

Methyl-3-[[3-(1-methoxyhexyl)phenoxy]methyl]benzoate

To a degreased suspension of sodium hydride (0.5 g) in ethyl ether at 0°C was added methyl-3-[[3-(1-hydroxyhexyl)phenoxy]methyl]benzoate (1.7 g) in ether. The mixture was allowed to warm to room temperature. Methyl iodide (0.6 ml) was added and the reaction was stirred at room temperature for 3 days. The reaction was quenched with saturated aqueous ammonium chloride. The mixture was extracted with ethyl ether. The organic extract was washed with water; dried $MgSO_4$) and concentrated to an oil. The oil was purified by HPLC on silica gel using a 1:9 ratio of ethyl acetate/hexanes as an eluent.

Example 5

Methyl-3-[[3-(1-tetrahydro-2H-pyran-2-yloxy)hexyl)phenoxy]methyl]benzoate

To a solution of methyl-3-[[3-(1-hydroxyhexyl)phenoxy]methyl]benzoate (2.2 g), dihydropyran (2.3 ml) and ethyl ether was added a catalytic amount of *para*-toluene sulfonic acid. The reaction was stirred at room temperature for 4 days. The ethyl ether was removed *in vacuo* and the remaining oil was purified by HPLC on silica gel using 7:93 ratio of ethyl acetate/hexanes as an eluent (1.8 g, 67% yield).

In like manner as above, using appropriate starting materials, the following compound was made:

Methyl-2-[[3-[1-(tetrahydro-2H-pyran-2-yloxy)pentyl]phenoxy]methyl]benzoate.

Example 6

2-[3-(1-hydroxypentyl)phenoxy]methylbenzenemethanol

To a suspension of lithium aluminum hydride (1.0 g) in ethyl ether (100 ml) at 0°C was added dropwise a solution of methyl-4-[[3-(1-hydroxyhexyl)phenoxy]methyl]benzoate (3.2 g) in ethyl ether (100 ml). The reaction was consecutively quenched with 1 ml of water, 1 ml of 15% sodium hydroxide and 3 ml of water. The mixture was filtered and the ethyl ether was removed *in vacuo* (1.9 g, 65% yield).

Example 7

3-[[3-(1-hydroxyhexyl)phenoxy]methyl]benzaldehyde

To a solution of 3-[[3-(1-hydroxyhexyl)phenoxy]methyl]benzonitrile (10.0 g) in THF was added diisobutylaluminum hydride (15 g) and the reaction was refluxed overnight. Methanol (10.2 ml) was slowly added followed by water (5.7 ml). The mixture was filtered and the filtrate was concentrated to an oil. The oil was dissolved in chloroform. The solution was washed with 5% aqueous hydrochloric acid (4 times); dried ($MgSO_4$) and concentrated to an oil (3.0 g, 30% yield).

In like manner as above, using appropriate starting materials, the following intermediate was prepared:

Phenyl-3-formylbenzyl ether.

Example 8

3-[[3-(1-hydroxyhexyl)phenoxy]methyl]benzyl amine hydrochloride

To a suspension of lithium aluminum hydride (1.0 g) in ethyl ether, was added dropwise a solution of 3-[[3-(1-hydroxyhexyl)phenoxy]methyl]benzonitrile (3.0 g) in ethyl ether. After stirring for 2 h at room temperature the reaction was quenched consecutively with 1 ml $H_2O$, 1 ml 15% sodium hydroxide and 3 ml

of water. The mixture was filtered. The remaining solution was treated with ethereal hydrochloric acid and a precipitate formed. The precipitate was filtered and dried, giving 2.2 g (63% yield) of solid, m.p. 90—94°C.

Example 9

3-[[3-(1-hydroxyhexyl)phenoxy]methyl]benzamide

A mixture of 3-[[3-(1-hydroxyhexyl)phenoxy]methyl]benzonitrile (5.0 g) 30% hydrogen peroxide (6.6 ml) ethanol (9 ml) and 6*N* sodium hydroxide (0.7 ml) was stirred at room temperature for 1 h. The reaction was then heated to 50°C for 3 h. The solution was neutralized with 5% aqueous hydrochloric acid and extracted with chloroform. The organic extract was dried ($MgSO_4$) and concentrated to a solid. The solid was recrystallized from ethyl acetate giving 3.4 g (63% yield) of solid, m.p. 97—98°C.

Example 10

Benzyl-3-(hexanoyl)phenyl ether

To a suspension of pyridinium chlorochromate (32.3 g) in methylene chloride (200 ml) was added a solution of benzyl-3-[1-hydroxy)hexyl]phenyl ether (28.4 g) in methylene chloride (25 ml). The reaction was stirred at room temperature for 1½ h. The excess methylene chloride was decanted and residual black solid was triturated with ethyl ether (four times). The combined organic extract was purified on fluorosil using ethyl ether as an eluent (27.5 g, 98% yield).

Example 11

2-[[3-(1-hydroxyhexyl)phenoxy]methyl]pyridine hydrochloride

A suspension of 3.3 g of picolyl chloride hydrochloride (Aldrich 16,270-1), 1-(3-hydroxyphenyl)-1-hexanol (3.9 g), cesium carbonate (16.3 g), cesium iodide (trace) and acetone was refluxed for 40 h. The reaction was filtered through a pad of celite and silica gel and the solvent was removed *in vacuo*. The remaining oil was dissolved in ethyl ether, filtered through celite and silica gel and treated with ethereal hydrochloric acid. The resulting white precipitate was filtered, washed (ethyl ether) and dried giving 4.2 g (66% yield) of solid, m.p. 164—165°C.

In like manner as above using appropriate starting materials and reagents, the following compounds were prepared:

3-[[3-(1-hydroxyhexyl)phenoxy]methyl]pyridine hydrochloride, m.p. 162—163°C;
2-[[3-(1-hydroxyhexyl)phenoxy]methyl]quinoline hydrochloride, m.p. 90—95°C;
4-[[3-(1-hydroxyhexyl)phenoxy]methyl]pyridine hydrochloride, m.p. 160—165.5°C.

Example 12

Methyl-6-[[3-(1-hydroxyhexyl)phenoxy]methyl]picolinate

A solution of 6-[[3-(1-hydroxyhexyl)phenoxy]methyl]picolinyl nitrile (1.1 g), methanol (50 ml) and cesium carbonate was stirred at room temperature overnight. The reaction mixture was diluted with 0.1*N* hydrochloric acid. After stirring for 3 h, the methanol was removed *in vacuo* and the remaining suspension was extracted with chloroform. The organic extract was dried ($MgSO_4$) and concentrated to an oil (7.6 g, 94% yield).

Example 13

Benzyl-3-[1-(N-methylamino)hexyl]phenyl ether

A solution of benzyl-3-(hexanoyl)phenyl ether (2.8 g) 40% aqueous methylamine (1.5 ml) and methanol adjusted to pH 6 with 5% aqueous hydrochloric acid is treated with a methanolic solution of sodium borohydride. The reaction is stirred overnight. The methanol is removed *in vacuo* and the remaining mixture is extracted with methylene chloride. The organic extract is dried ($MgSO_4$) and concentrated to an oil.

In like manner as above, using appropriate starting materials the following compounds can be prepared:

3-chlorobenzyl-3-[1-(*n*-butylamino)hexyl]phenyl ether; and
2-trifluoromethyl-3-[1-(N,N-dimethylamino)hexyl]phenyl ether.

Example 14

Benzyl-3-[1-hydroxyl-2,2-(dimethyl)hexyl]phenyl ether

To a solution of lithium amide (0.36 g) and methyl iodide (3.02 g) in THF (10 ml) at reflux is slowly added a solution of benzyl-3-(hexanoyl)phenyl ether (2.0 g) in THF (10 ml). The reaction is heated at reflux for 2 h. The THF is removed *in vacuo*. The residue is dissolved in ethyl acetate, washed with water and brine; dried ($MgSO_4$) and concentrated to an oil. The oil is dissolved in ether and slowly added to a suspension of lithium aluminum hydride (0.1 g) in ethyl ether. The reaction is then heated at reflux for 2 h. The reaction is quenched by consecutive treatment with water (0.1 ml), 1*N* NaOH (0.3 ml), water (0.1 ml). The mixture is filtered and the ethyl ether is removed *in vacuo* giving the desired oil.

In like manner as above, using appropriate starting materials and reagents, the following compounds can be prepared:

2,4-dibromobenzyl-3-[1-(hydroxyl)2-(isobutyl)hexyl]phenyl ether; and
4-fluorobenzyl-3-[1-(hydroxyl)2-(diethyl)heptyl]phenyl ether.

Example 15

3-(1-hydroxyhexyl)benzyl alcohol

To a solution of pentyl magnesium bromide (0.082 mol) in ethyl ether (100 ml) at 0°C as prepared in Example 2 is added cadmium chloride (8.06 g) portionwise. The suspension is stirred overnight at room temperature. The solvent is distilled and toluene (300 ml) is added. The mixture is refluxed for 1 h and cooled to room temperature. A solution of 3-carbomethoxy benzoyl chloride (48 g) in toluene (50 ml) is slowly added. The reaction is refluxed for 2 h. After cooling, 3% aqueous hydrochloric acid is added. The organic phase is separated and the aqueous phase is extracted with ethyl acetate. The combined extracts are dried ($MgSO_4$) and concentrated to an oil. The oil is dissolved in ethyl ether and slowly added to a suspension of lithium aluminum hydride (4.0 g) in ethyl ether. The reaction is heated at reflux for 2 h. The reaction was quenched by consecutive treatment with water (4 ml), 1*N* NaOH (12 ml) and water (4 ml). The mixture is filtered and the ethyl ether is removed *in vacuo* giving the desired oil.

Example 16

Tolyl-3-(1-hydroxyhexyl)benzyl sulfonate

To a solution of 3-(1-hydroxyhexyl)benzyl alcohol (20.8 g) in pyridine (50 ml) is added *para*-toluene sulfonyl chloride (20.1 g). The reaction is stirred at room temperature for 2 days. Ice is added and the mixture is extracted with ethyl ether (twice). The organic extract is washed with 5% aqueous hydrochloric acid (four times), brine; dried ($MgSO_4$) and concentrated to an oil.

Example 17

Methyl-3-[[3-(1-hydroxyhexyl)anilinyl]methyl]benzoate

A mixture of tolyl-3-(3-(1-hydroxyhexyl)benzyl sulfonate (3.6 g), methyl-3-aminobenzoate (1.5 g) (Pfaltz and Bauer M10720), cesium carbonate (3.2 g), and acetone (200 ml) is refluxed for 2 days. The reaction is filtered and concentrated to an oil. The oil is purified by HPLC on silica gel.

In like manner as above, using appropriate starting materials, and reagents, the following compounds can be prepared:

Ethyl-2-[[3-(1-hydroxybutyl)anilinyl]methyl]benzoate;
Isopropyl-4-[[3-(1-hydroxybutyl)anilinyl]methyl]benzoate;
Methyl-3-[[2-(1-hydroxyhexyl)anilinyl]methyl]benzoate;
Phenyl-3-[1-hydroxy)hexyl]benzyl thioether;
3-Methoxyphenyl-3-[1-(hydroxyl)hexyl]benzyl thioether;
4-Nitrophenyl-3-[1(hydroxyl)hexyl]benzyl thioether; and
Methyl-2-[[3-(1-hydroxyhexyl)thiophenoxy]methyl benzoate.

The compounds of the present invention have potent activity in regulating the formation of lipoxygenase and as such possess therapeutic value in the treatment of inflammatory conditions and allergic responses such as anaphylaxis and asthma.

Lipoxygenases in mammals have been found in the lung, platelets, and white cells. They are enzymes capable of oxidizing arachiodonic acid into hydroperoxyeicosatetraenoic acids (HPETEs) and their stable products hydroxyeicosatetetraenoic acids ((HETEs). Lipoxygenases are classified according to the position in the arachidonic acid which is oxygenated. Platelets metabolize arachidonic acid to 12-HETE, while polymorphonuclear leukocytes contain 5 and 15 lipoxygenases. It is known that 12-HETE and 5, 12-diHETE are chemotactic for human neutrophilis and eosinophils, and may augment the inflammation process. 5-HPETE is known to be a precursor of slow-reacting substance of anaphylaxis (SRS-A). The SRS family of molecules, such leukotrienes B, C, and D, have been shown to be potent bronchoconstrictors (see NATURE *288,* 484—486 (1980)).

The following protocol describes as assay to detect inhibitors of the lipoxygenase pathway. Such inhibitors are believed to be capable of modulating the biosynthesis of the leukotrienes, a property believed to be useful in treating asthma and inflammatory disease states.

Protocol

A suspension of rat neutrophils in buffer is incubated for 3 min at 30°C with [$^{14}$C]-arachiodonic acid (AA) and Calcium Ionophore A23187. Citric acid (2M) is used to quench the reaction. Following the addition of a trace amount of ($^{3}$H)-5-HETE together with an excess of unlabelled 5-HETE to each tube, the mixture is extracted with chloroform/methanol. The organic layer is washed with dilute acid and an aliquot is transferred to glass tubes and dried. The residue is dissolved in a small volume of chloroform and an aliquot is spotted on silica gel TLC sheets, which are developed with an ethyl acetate/isoctane/water/acetic acid solvent system. The 5-HETE spots are visualized with iodine, cut out and placed in scintillation vials for counting. After adjusting for the extraction efficiency, the amount (pmole) of [$^{14}$C]-5-HETE in each of the tubes is quantitated. The net pmoles of 5-HETE are obtained by subtracting the pmoles of 5-HETE in the tubes containing buffer alone (blank) from the pmoles of 5-HETE in the tubes containing buffer and cells

(control). The ability of the test compounds to modulate the activity of this enzyme is determined by a decrease or increase in the net amount of 5-HETE produced.

In Table I and Table II, the last column shows the concentration required for 50% inhibition of the 5-lipoxygenase pathway (5-LOX/$I_{50}$M) for some representative compounds according to the present invention.

TABLE I

Aryleicosanoids as 5-Lipoxygenase Inhibitors

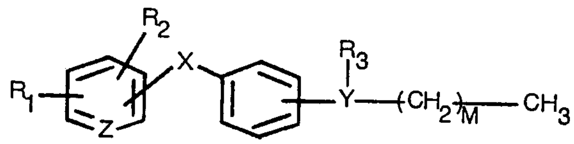

(I)

| No. | $R_1$ | $R_2$ | Z | X | Y | $R_3$ | M | 5-LOX $I_{50}\mu M$ |
|---|---|---|---|---|---|---|---|---|
| 1 | 3-$CO_2H$ | H | C | $CH_2O$ | 3-$CHCH_2$ | OH | 3 | 25 |
| 2 | 2-$CO_2H$ | H | C | $CH_2O$ | 3-$CHCH_2$ | OH | 3 | 30 |
| 3 | 2-$CO_2H$ | H | C | $CH_2O$ | 3-$CHCH_2$ | OH | 2 | 10 |
| 4 | 4-$CO_2H$ | H | C | $CH_2O$ | 3-$CHCH_2$ | OH | 3 | 21 |
| 5 | 3-$CO_2CH_3$ | H | C | $CH_2O$ | 3-$CHCH_2$ | OH | 3 | 1.2 |
| 6 | 2-$CO_2CH_3$ | H | C | $CH_2O$ | 3-$CHCH_2$ | OH | 3 | 0.6 |
| 7 | 2-$CO_2CH_3$ | H | C | $CH_2O$ | 3-$CHCH_2$ | OH | 2 | 3.0 |
| 8 | 4-$CO_2CH_3$ | H | C | $CH_2O$ | 3-$CHCH_2$ | OH | 3 | 3.2 |
| 9 | 3-$CO_2CH_3$ | H | C | $CH_2O$ | 2-$CHCH_2$ | OH | 3 | 3.2 |
| 10 | 3-$CH_2CO_2H$ | H | C | $CH_2O$ | 3-$CHCH_2$ | OH | 3 | 2.5 |
| 11 | 3-$CH_2CO_2CH_3$ | H | C | $CH_2O$ | 3-$CHCH_2$ | OH | 3 | 5.8 |
| 12 | 3-$CO_2CH_3$ | H | C | $CH_2O$ | 3-$CHCH_2$ | $OCOCH_3$ | 3 | 5.0 |
| 13 | 3-$CO_2CH_3$ | H | C | $CH_2O$ | 3-$CHCH_2$ | $OCH_3$ | 3 | 10 |
| 14 | 3-$CO_2CH_3$ | H | C | $CH_2O$ | 3-$CHCH_2$ | OTHP | 3 | 23 |
| 15 | 2-$CO_2CH_3$ | H | C | $CH_2O$ | 3-$CHCH_2$ | OTHP | 2 | 10 |
| 16 | 2-$CH_2OH$ | H | C | $CH_2O$ | 3-$CHCH_2$ | OH | 3 | 4 |

TABLE II
Aryleicosanoids as 5-Lipoxygenase Inhibitors

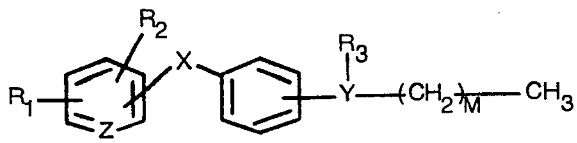

(I)

| No. | $R_1$ | $R_2$ | Z | X | Y | $R_3$ | M | 5-$LOX_3$ $I_{50}\mu M$ |
|---|---|---|---|---|---|---|---|---|
| 17 | 3-CHO | H | C | $CH_2O$ | 3-$CHCH_2$ | OH | 2 | 4.0 |
| 18 | 3-CN | H | C | $CH_2O$ | 3-$CHCH_2$ | OH | 3 | 17 |
| 19 | 3-$CH_2NH_2$ | H | C | $CH_2O$ | 3-$CHCH_2$ | OH | 3 | — |
| 20 | 3-$CONH_2$ | H | C | $CH_2O$ | 3-$CHCH_2$ | OH | 3 | 2.0 |
| 21 | 4-$OCH_2CO_2CH_3$ | H | C | $CH_2O$ | 3-$CHCH_2$ | OH | 3 | 4.8 |
| 22 | H | H | C | $CH_2O$ | 3-$CHCH_2$ | OH | 3 | 2.7 |
| 23 | H | H | C | $OCH_2$ | 3-$CHCH_2$ | OH | 3 | 6.7 |
| 24 | 3-$CO_2CH_3$ | H | C | $CH_2O$ | 3-CH=CH | $CH_3$ | 3 | 5.0 |
| 25 | 3-$CO_2CH_3$ | H | C | $CH_2O$ | 4-CH=CH | H | 3 | 100 |
| 26 | H | H | C | $CH_2O$ | 3-C-$CH_2$ | O | 3 | 2.0 |
| 27 | H | H | N | 4-$CH_2O$ | 3-$CHCH_2$ | OH | 3 | 2.7 |
| 28 | H | H | N | 2-$CH_2O$ | 3-$CHCH_2$ | OH | 3 | 0.5 |
| 29 | H | H | N | 3-$CH_2O$ | 3-$CHCH_2$ | OH | 3 | 2.7 |
| 30 | 3-CN | H | N | 2-$CH_2O$ | 3-$CHCH_2$ | OH | 3 | 30 |
| 31 | 3-$CO_2CH_3$ | H | N | 2-$CH_2O$ | 3-$CHCH_2$ | OH | 3 | 3.0 |
| 32 | 3-$CO_2H$ | H | N | 2-$CH_2O$ | 3-$CHCH_2$ | OH | 3 | 6.0 |
| 33 | 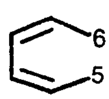 | H | N | 2-$CH_2O$ | 3-$CHCH_2$ | OH | 3 | 0.3 |

The therapeutic compounds of this invention may be administered to a mammal alone or in combination with pharmaceutically acceptable carriers, the proportion of which is determined by the solubility and chemical nature of the compound, chosen route of administration and standard pharmaceutical practice.

The physician will determine the dosage of the present therapeutic agents which will be most suitable and it will vary with the form of administration and the particular compound chosen and also, it will vary with the particular patient under treatment. He will generally wish to initiate treatment with small dosages substantially less than the optimum dose of the compound and increase the dosage by small increments until the optimum effect under the circumstances is reached. The therapeutic dosage will generally be from 0.1 to 100 μM per day and higher although it may be administered in several different dosage units.

**Claims for the Contracting States: BE CH DE FR GB IT LI LU NL SE**

1. Compounds of the general formula I

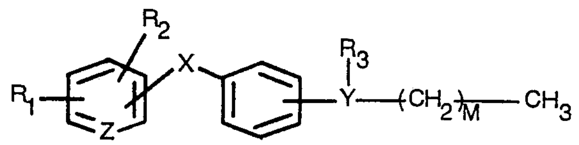

(I)

and pharmaceutically acceptable salts thereof, wherein

$R_1$ and $R_2$ are independently hydrogen, alkyl, carboxy, carboxyalkyl, carbalkoxyalkyl, carbalkoxy, formyl, amino, aminoalkyl, alkylamino, carboxamide, halo, trihalomethyl, hydroxy, or $R_1$ and $R_2$ when taken together with the carbon atoms to which they are attached form a phenyl ring;

Z is CH or N;

X is —O—, $—CH_2Z_1—$, —HC=CH—, —C≡C— or $—CH_2Z_1CH_2$;

Y is $—CHCH_2—$, —C=CH—, $—CHCHR_5$ or $—CHC(R_5)_2—$ when $R_3$ is monovalent or $—C—CH_2$ when $R_3$ is O;

$R_3$ is O, OH, $OR_4$, NH, $HNR_4$ or $N(R_4)_2$; and

M is an integer from 0 to 5;

wherein $Z_1$ is O, S or $NR_4$; $R_4$ is H, alkyl, pyranyl or aryl; and $R_5$ is H, alkyl or fluorine, with the proviso that when $R_3$ is OH, M is 3 and the relationship of X and Y is meta or para then one of $R_1$ or $R_2$ is other than hydrogen.

2. The compounds of claim 1 of the formula:

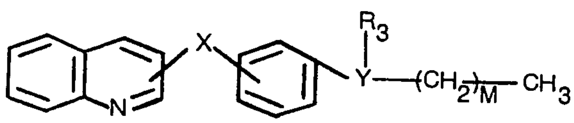

and pharmaceutically acceptable salts thereof, wherein X, Y, $R_3$ and M are as defined in claim 1.

3. The compounds of claim 1 or 2 wherein the carboxyalkyl, carbalkoxyalkyl, alkylamino, aminoalkyl or carbalkoxy groups contain up to 6 carbon atoms.

4. The compounds of claim 1 or 2 wherein the halo group in halo and trihalomethyl is F, Cl, Br or I.

5. The compounds of claim 1 or 2 wherein the aryl group in $R_4$ is phenyl or naphthyl.

6. The compounds of claim 1 or 2 and pharmaceutically acceptable salts thereof, wherein

$R_1$ and $R_2$ are independently H, carboxy, carboxyalkyl, carbalkoxy or $R_1$ and $R_2$ form a phenyl ring with the carbon atoms to which they are attached;

X is —CH=CH— or $—CH_2Z_1$;

Y is $—CHCH_2$ or $—CHC(R_5)_2—$;

$R_3$ is OH or $HNR_4$; and

M is an integer from 1 to 5.

7. The compounds of claim 1 or 2 wherein $R_4$ is H, methyl, ethyl, propyl or phenyl.

8. The compounds of claims 1 or 2 and pharmaceutically acceptable salts thereof, wherein

$R_1$ and $R_2$ form a phenyl ring with the carbon atoms to which they are attached;

X is $CH_2Z_1$;

Y is $CHCH_2$;

$R_3$ is OH or $HNR_4$; and

M is an integer from 2 to 3; and

$Z_1$ is O.

9. Methyl-3-[[3-(1-hydroxyhexyl)phenoxy]methyl]benzoate.

10. Methyl-2-[[3-(1-hydroxyhexyl)phenoxy]methyl]benzoate.

11. 2-[[3-(1-hydroxyhexyl)phenoxy]methyl]pyridine.

12. 2-[[3-(1-hydroxyhexyl)phenoxy]methyl]quinoline and pharmaceutically acceptable salts thereof.

13. Compounds of the general formula I of claim 1 wherein

$R_1$ is $3-CO_2CH_3$, $R_2$ is hydrogen, Z is CH, X is $CH_2O$, Y is $3-CHCH_2$, $R_3$ is $OCOCH_3$ or tetrahydropyranoyl and M is 3; or

$R_1$ is $2-CO_2CH_3$, $R_2$ is hydrogen, Z is CH, X is $3-CHCH_2$, $R_3$ is OH and M is 3; or

$R_1$ and $R_2$ are hydrogen, Z is CH, X is $OCH_2$,

Y is $3-CHCH_2$, $R_3$ is OH and M is 3; or

$R_1$ is $3-CO_2CH_3$, $R_2$ is hydrogen, Z is CH, X is $CH_2O$, Y is 3-CH=CH, $R_3$ is $CH_3$ and M is 3; or

$R_1$ is $3-CO_2CH_3$, $R_2$ is hydrogen, Z is CH, X is $CH_2O$, Y is 4-CH=CH, $R_3$ is hydrogen and M is 3.

14. A therapeutic composition comprising as an active ingredient a compound of any of claims 1 to 13.

15. A process for the production of a compound of any of claims 1 to 12 which includes at least one of the following:

(a) condensation of a compound of formula II and III

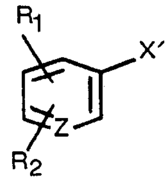

(II)

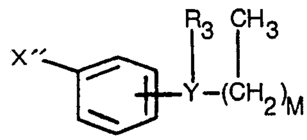

(III)

wherein X′ and X″ are —$CH_2$Hal, Hal, OH, $CH_2OH$, SH or $CH_2SH$,

(b) condensation of a compound of the above formula II and III wherein X′ and X″ are —CH=$CH_2$, —C≡CH or Hal;

(c) condensation of a compound of the above formula II and III wherein X′ and X″ are —CHO or —$NH_2$;

(d) condensation of a compound of the above formula II and III wherein X′ and X″ are COOH, or $Z_1$H;

(e) condensation of a compound of the above formula II and III wherein X′ and X″ are H or an acylating group;

(f) condensation of a compound of the above formula II and III wherein X′ and X″ are —$NR_4$H or halogen;

(g) acylation or alkylation of a compound of formula IV

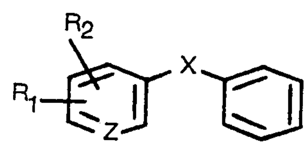

(IV)

to introduce the substituent —Y($R_3$)—$(CH_2)_M CH_3$; and optionally forming salts especially pharmaceutically acceptable salts of the salt forming products obtained thereby; and optionally introducing substituents $R_1$ and $R_2$ by known substitution reactions.

**Claims for the Contracting State: AT**

1. A process for the production of compounds of the general formula I

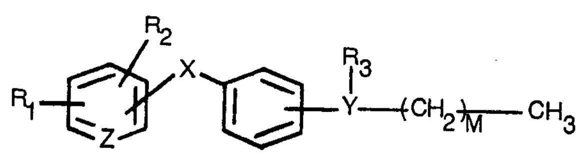

(I)

and pharmaceutically acceptable salts thereof, wherein

$R_1$ and $R_2$ are independently hydrogen, alkyl, carboxy, carboxyalkyl, carbalkoxyalkyl, carbalkoxy, formyl, amino, aminoalkyl, alkylamino, carboxamide, halo, trihalomethyl, hydroxy, or $R_1$ and $R_2$ when taken together with the carbon atoms to which they are attached form a phenyl ring;

Z is CH or N;

X is —O—, —$CH_2Z_1$—, —HC=CH—, —C≡C— or —$CH_2Z_1CH_2$;

Y is —$CHCH_2$—, —C=CH—, —$CHCHR_5$ or —$CHC(R_5)_2$— when $R_3$ is monovalent or —C—$CH_2$ when $R_3$ is O;

$R_3$ is O, OH, $OR_4$, NH, $HNR_4$ or $N(R_4)_2$; and

M is an integer from 0 to 5;

wherein $Z_1$ is O, S or $NR_4$; $R_4$ is H, alkyl, pyranyl or aryl; and $R_5$ is H, alkyl or fluorine, with the proviso that when $R_3$ is OH, M is 3 and the relationship of X and Y is meta or para then one of $R_1$ or $R_2$ is other than hydrogen, which comprises at least one of the following steps:

(a) condensation of a compound of formula II

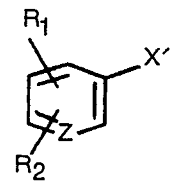

(II)

and III

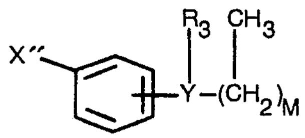

(III)

wherein X′ and X″ are —$CH_2$Hal, Hal, OH, $CH_2OH$, SH or $CH_2SH$,

(b) condensation of a compound of the above formula II and III wherein X′ and X″ are —$CH{=}CH_2$, —$C{\equiv}CH$ or Hal;

(c) condensation of a compound of the above formula II and III wherein X′ and X″ are —CHO or —$NH_2$;

(d) condensation of a compound of the above formula II and III wherein X′ and X″ are COOH, or $Z_1H$;

(e) condensation of a compound of the above formula II and III wherein X′ and X″ are H or an acylating group;

(f) condensation of a compound of the above formula II and III wherein X′ and X″ are —$NR_4H$ or halogen;

(g) acylation or alkylation of a compound of formula IV

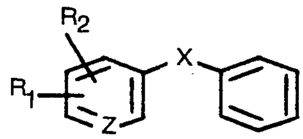

(IV)

to introduce the substituent —$Y(R_3)$—$(CH_2)_MCH_3$; and optionally forming salts especially pharmaceutically acceptable salts of the salt forming products obtained thereby; and optionally introducing substituents $R_1$ and $R_2$ by known substitution reactions.

2. The process of claim 1 wherein the product is a compound of the formula

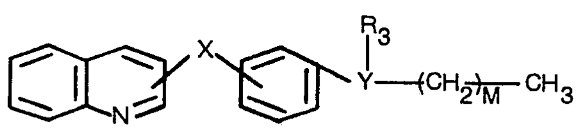

and pharmaceutically acceptable salts thereof, wherein X, Y, $R_3$ and M are as defined in claim 1.

3. The process of claim 1 or 2 wherein the carboxyalkyl, carbalkoxyalkyl, alkylamino, aminoalkyl or carbalkoxy groups contain up to 6 carbon atoms.

4. The process of claim 1 or 2 wherein the halo group in halo and trihalomethyl is F, Cl, Br or I.

5. The process of claim 1 or 2 wherein the aryl group in $R_4$ is phenyl or naphthyl.

6. The process of claim 1 or 2 wherein

$R_1$ and $R_2$ are independently H, carboxy, carboxyalkyl, carbalkoxy or $R_1$ or $R_2$ form a phenyl ring with the carbon atoms to which they are attached;

X is —$CH{=}CH$— or —$CH_2Z_1$;

Y is —$CHCH_2$ or —$CHC(R_5)_2$—;

$R_3$ is OH or $HNR_4$; and

M is an integer from 1 to 5.

7. The process of claim 1 or 2 wherein $R_4$ is H, methyl, ethyl, propyl or phenyl.

8. The process of claim 1 or 2 wherein

$R_1$ and $R_2$ form a phenyl ring with the carbon atoms to which they are attached;

X is $CH_2Z_1$;

Y is $CHCH_2$;

$R_3$ is OH or $HNR_4$;

M is an integer from 2 to 3; and

$Z_1$ is O.

9. The process of claim 1 wherein the product is Methyl-3-[[3-(1-hydroxyhexyl)phenoxy]-methyl]benzoate.

10. The process of claim 1 wherein the product is Methyl-2-[[3-(1-hydroxyhexyl)phenoxy]-methyl]benzoate.

11. The process of claim 1 wherein the product is 2-[[3-(1-hydroxyhexyl)phenoxy]methyl]pyridine.

12. The process of claim 1 wherein the product is 2-[[3-(1-hydroxyhexyl)phenoxy]methyl]quinoline and pharmaceutically acceptable salts thereof.

13. A process for the production of a therapeutic composition comprising as an active ingredient a compound with any of claims 1 to 12 and pharmaceutically acceptable carriers and/or diluents.

**Patentansprüche für die Vertragsstaaten: BE CH DE FR GB IT LI LU NL SE**

1. Verbindungen der allgemeinen Formel I

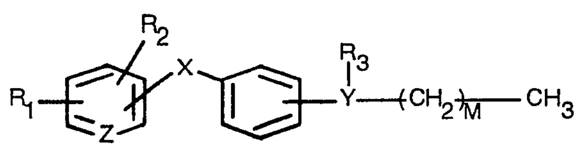

(I)

und pharmazeutisch annehmbare Salze hiervon, worin bedeuten:

$R_1$ und $R_2$ unabhängig voneinander Wasserstoff, Alkyl, Carboxy, Carboxyalkyl, Carbalkoxyalkyl, Carbalkoxy, Formyl, Amino, Aminoalkyl, Alkylamino, Carboxamid, Halogen, Trihalogenmethyl, Hydroxy oder

$R_1$ oder $R_2$ bilden, wenn sie mit den Kohlenstoffatomen, an die sie gebunden sind, zusammengenommen werden, einen Phenylring;

Z CH oder N;

X —O—, $—CH_2Z_1$, —HC=CH—, —C≡C— oder $—CH_2Z_1CH_2—$;

Y $—CHCH_2—$, —C=CH—, $—CHCHR_5$ oder $—CHC(R_5)_2—$, wenn $R_3$ monovalent ist oder $—C—CH_2$, wenn $R_3$ O ist;

$R_3$ O, OH, $OR_4$, NH, $NHR_4$ oder $(N(R_4)_2$; und

M eine ganze Zahl von 0 bis 5;

worin $Z_1$, O, S oder $NR_4$; $R_4$ H, Alkyl, Pyranyl oder Aryl; und $R_5$ H, Alkyl oder Fluor bedeuten, mit der Maßgabe, daß , wenn $R_3$ OH ist, M 3 ist und die Stellung von X und Y meta oder para ist, dann eines von $R_1$ oder $R_2$ eine andere Bedeutung als Wasserstoff hat.

2. Verbindungen nach Anspruch 1 der Formel:

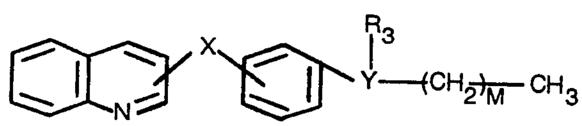

und pharmazeutisch annehmbare Salze hiervon, wobei X, Y, $R_3$ und M wie in Anspruch 1 definiert sind.

3. Verbindungen nach Anspruch 1 oder 2, wobei die Carboxyalkyl-, Carbalkoxyalkyl-, Alkylamino-, Aminoalkyl- oder Carbalkoxygruppen bis zu 6 Kohlenstoffatome enthalten.

4. Verbindungen nach Anspruch 1 oder 2, wobei die Halogengruppe in Halogen und Trihalogenmethyl F, Cl, Br oder J ist.

5. Verbindungen nach Anspruch 1 oder 2, wobei die Arylgruppe in $R_4$ Phenyl oder Naphthyl ist.

6. Verbindungen nach Anspruch 1 oder 2 und pharmazeutisch annehmbare Salze hiervon, wobei

$R_1$ und $R_2$ unabhängig voneinander H, Carboxy, Carboxyalkyl, Carbalkoxy sind oder $R_1$ und $R_2$ mit den Kohlenstoffatomen, an die sie gebunden sind, einen Phenylring bilden;

X —CH=CH— oder $—CH_2Z_1$ ist;

Y $—CHCH_2$ oder $—CHC(R_5)_2—$ ist;

$R_3$ —OH oder $HNR_4$ ist; und

M eine ganze Zahl von 1 bis 5 ist.

7. Verbindungen nach Anspruch 1 oder 2, wobei $R_4$ H, Methyl, Ethyl, Propyl oder Phenyl ist.

8. Verbindungen nach Anspruch 1 oder 2 und pharmazeutisch annehmbare Salze hiervon, wobei

$R_1$ und $R_2$ mit den Kohlenstoffatomen, an die sie gebunden sind, einen Phenylring bilden;

X $—CH_2Z_1$ ist;

Y $—CHCH_2$ ist;

$R_3$ —OH oder $HNR_4$ ist; und

M eine ganze Zahl von 2 bis 3 ist; und

$Z_1$ 0 ist.

9. Methyl-3-[[3-(1-hydroxyhexyl)phenoxy]-methyl]-benzoat.
10. Methyl-2-[[3-(1-hydroxyhexyl)phenoxy]-methyl]benzoat.
11. 2-[[3-(1-hydroxyhexyl)phenoxy]methyl]-pyridin.
12. 2-[[3-(1-hydroxyhexyl)phenoxy]methyl]-chinolin und pharmazeutisch annehmbare Salze hiervon.
13. Verbindungen der allgemeinen Formel I nach Anspruch 1, wobei
$R_1$ 3-$CO_2CH_3$ ist, $R_2$ Wasserstoff ist, Z CH ist, X $CH_2O$ ist, Y 3-$CHCH_2$ ist, $R_3$ $OCOCH_3$ oder Tetrahydropyranoyl ist und M 3 ist; oder
$R_1$ 2-$CO_2CH_3$ ist, $R_2$ Wasserstoff ist, Z CH ist, X 3-$CHCH_2$ ist, $R_3$ OH ist und M 3 ist; oder
$R_1$ und $R_2$ Wasserstoff sind, Z CH ist, X $OCH_2$ ist, Y 3-$CHCH_2$ ist, $R_3$ OH ist und M 3 ist; oder
$R_1$ 3—$CO_2CH_3$ ist, $R_2$ Wasserstoff ist, Z CH ist, X $CH_2$ O ist, Y 3-CH=CH ist, $R_3$ $CH_3$ ist und M 3 ist; oder
$R_1$ 3—$CO_2CH_3$ ist, $R_2$ Wasserstoff ist, Z CH ist, X $CH_2O$ ist, Y 4-CH=CH ist, $R_3$ Wasserstoff ist und M 3 ist.
14. Therapeutische Zusammensetzung, die als Wirkstoff eine Verbindung nach mindestens einem der Ansprüche 1 bis 13 enthält.
15. Verfahren zur Herstellung einer Verbindung nach mindestens einem der Ansprüche 1 bis 12, umfassend mindestens eines der folgenden:
(a) Kondensation einer Verbindung der Formeln II und III

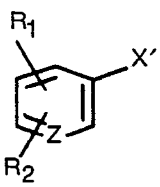

(II)

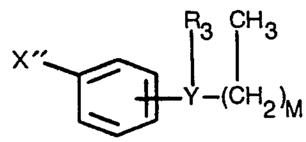

(III)

worin X' und X'' —$CH_2$Hal, Hal, OH, $CH_2OH$, SH oder $CH_2SH$ sind,
(b) Kondensation einer Verbindung der obigen Formeln II und III, worin X' und X'' —CH=$CH_2$, —C≡CH oder Halogen sind;
(c) Kondensation einer Verbindung der obigen Formeln II und III, worin X' und X'' —CHO oder $NH_2$ sind;
(d) Kondensation einer Verbindung der obigen Formeln II und III, worin X' und X'' —COOH oder $Z_1H$ sind;
(e) Kondensation einer Verbindung der obigen Formeln II und III, worin X' und X'' H oder eine Acylierungsgruppe sind;
(f) Kondensation einer Verbindung der obigen Formeln II und III, worin X' und X'' —$NR_4H$ oder Halogen sind;
(g) Acylierung oder Alkylierung einer Verbindung der Formel (IV)

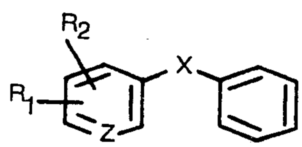

(IV)

um den Substituenten —$Y(R_3)$-$(CH_2)_MCH_3$ einzuführen; und wahlweise Bilden von Salzen, insbesondere pharmazeutisch annehmbaren Salzen der dadurch erhaltenen salzbildenden Produkte; und wahlweise Einführen der Substituenten $R_1$ und $R_2$ durch bekannte Substitutionsreaktionen.

**Patentansprüche für den Vertragsstaat: AT**

1. Verfahren zur Herstellung von Verbindungen der allgemeinen Formel (I)

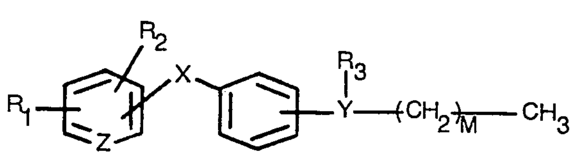

(I)

und pharmazeutisch annehmbare Salzen hiervon, worin bedeuten:

$R_1$ und $R_2$ unabhängig voneinander Wasserstoff, Alkyl, Carboxy, Carboxyalkyl, Carbalkoxyalkyl, Carbalkoxy, Formyl, Amino, Aminoalkyl, Alkylamino, Carboxamid, Halogen, Trihalogenmethyl, Hydroxy oder

$R_1$ oder $R_2$ bilden, wenn sie mit den Kohlenstoffatomen, an die sie gebunden sind, zusammengenommen werden, einen Phenylring;

Z CH oder N;

X —O—, $—CH_2Z_1$, —HC=CH—, —C≡C— oder $—CH_2Z_1CH_2—$;

Y $—CHCH_2—$, —C=CH—, $—CHCHR_5$ oder $—CHC(R_5)_2—$, wenn $R_3$ monovalent ist oder $—C—CH_2$, wenn $R_3$ O ist;

$R_3$ O, OH, $OR_4$, NH, $NHR_4$ oder $(N(R_4)_2$; und

M eine ganze Zahl von 0 bis 5;

worin $Z_1$, O, S oder $NR_4$; $R_4$ H, Alkyl, Pyranyl oder Aryl; und $R_5$ H, Alkyl oder Fluor bedeuten, mit der Maßgabe, daß, wenn $R_3$ OH ist, M 3 ist und die Stellung von X und Y meta oder para ist, dann eines von $R_1$ oder $R_2$ eine andere Bedeutung als Wasserstoff hat,

umfassend mindestens eine der folgenden Stufen:

(a) Kondensation einer Verbindung der Formeln II und III

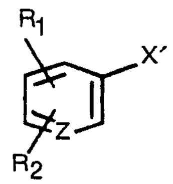

(II).

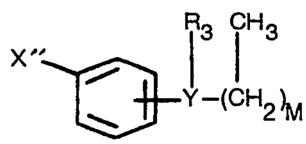

(III)

worin X′ und X″ $—CH_2Hal$, Hal, OH, $CH_2OH$, SH oder $CH_2SH$ sind,

(b) Kondensation einer Verbindung der obigen Formeln II und III, worin X′ und X″ $—CH=CH_2$, —C≡CH oder Halogen sind;

(c) Kondensation einer Verbindung der obigen Formeln II und III, worin X′ und X″ —CHO oder $NH_2$ sind;

(d) Kondensation einer Verbindung der obigen Formeln II und III, worin X′ und X″ —COOH oder $Z_1H$ sind;

(e) Kondensation einer Verbindung der obigen Formeln II und III, worin X′ und X″ H oder eine Acylierungsgruppe sind;

(f) Kondensation einer Verbindung der obigen Formeln II und III, worin X′ und X″ $—NR_4H$ oder Halogen sind;

(g) Acylierung oder Alkylierung einer Verbindung der Formel (IV)

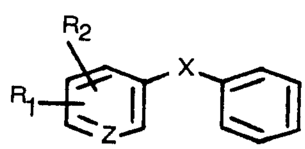

(IV)

um den Substituenten $—Y(R_3)\text{-}(CH_2)_MCH_3$ einzuführen; und wahlweise Bilden von Salzen, insbesondere pharmazeutisch annehmbaren Salzen der dadurch erhaltenen salzbildenden Produkte; und wahlweise Einführen der Substituenten $R_1$ und $R_2$ durch bekannte Substitutionsreaktionen.

2. Verfahren nach Anspruch 1, wobei das Produkt eine Verbindung der Formel ist

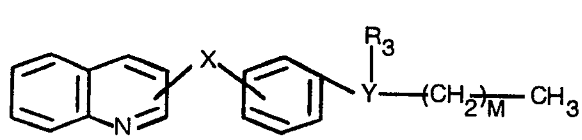

und pharmazeutisch annehmbare Salze hiervon, wobei X, Y, $R_3$ und M wie in Anspruch 1 definiert sind.

3. Verfahren nach Anspruch 1 oder 2, wobei die Carboxyalkyl-, Carbalkoxyalkyl-, Alkylamino-, Aminoalkyl- oder Carbalkoxygruppen bis zu 6 Kohlenstoffatome enthalten.

4. Verfahren nach Anspruch 1 oder 2, wobei die Halogengruppe in Halogen und Trihalogenmethyl F, Cl, Br oder J ist.

5. Verfahren nach Anspruch 1 oder 2, wobei die Arylgruppe in $R_4$ Phenyl oder Naphthyl ist.

6. Verfahren nach Anspruch 1 oder 2, worin $R_1$ und $R_2$ unabhängig voneinander H, Carboxy, Carboxyalkyl, Carbalkoxy sind oder $R_1$ und $R_2$ mit den Kohlenstoffatomen, an die sie gebunden sind, einen Phenylring bilden;

X —CH=CH— oder —$CH_2Z_1$ ist;

Y —$CHCH_2$ oder —$CHC(R_5)_2$— ist;

$R_3$ —OH oder $HNR_4$ ist; und

M eine ganze Zahl von 1 bis 5 ist.

7. Verfahren nach Anspruch 1 oder 2, wobei $R_4$ H, Methyl, Ethyl, Propyl oder Phenyl ist.

8. Verfahren nach Anspruch 1 oder 2, wobei $R_1$ und $R_2$ unabhängig voneinander Wasserstoff sind oder $R_1$ und $R_2$ mit den Kohlenstoffatomen, an die sie gebunden sind, einen Phenylring bilden;

X —$CH_2Z_1$ ist;

Y —$CHCH_2$ ist;

$R_3$ —OH oder $HNR_4$ ist;

M eine ganze Zahl von 2 bis 3 ist; und

$Z_1$ 0 ist.

9. Verfahren nach Anspruch 1, wobei das Produkt Methyl-3[[3-(1-hydroxyhexyl)phenoxy]-methyl]-benzoat ist.

10. Verfahren nach Anspruch 1, wobei das Produkt Methyl-2[[3-(1-hydroxyhexyl)phenoxy]-methyl]benzoat.

11. Verfahren nach Anspruch 1, wobei das Produkt 2-[[3-(1-hydroxyhexyl)phenoxy]methyl]-pyridin ist.

12. Verfahren nach Anspruch 1, wobei das Produkt 2-[[3-(1-hydroxyhexyl)phenoxy]methyl]-chinolin ist und pharmazeutisch annehmbare Salze hiervon.

13. Verfahren zur Herstellung einer therapeutischen Zusammensetzung, die als Wirkstoff eine Verbindung nach mindestens einem der Ansprüche 1 bis 12 sowie pharmazeutisch annehmbare Träger und/oder Verdünnungsmittel umfaßt.

**Revendications pour les Etats contractants: BE CH DE FR GB IT LI LU NL SE**

1. Composés de formule générale I:

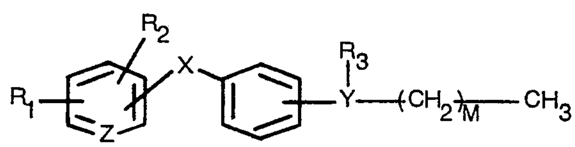

(I)

et leurs sels pharmaceutiquement acceptables, formule dans laquelle

$R_1$ et $R_2$ représentent, d'une manière indépendante, un hydrogène, un alkyle, un carboxy, un carboxyalkyle, un carbalkoxyalkyle, un carbalkoxy, un formyle, un amino, un aminoalkyle, un alkylamino, un carboxamide, un halogène, un trihalométhyle, un hydroxy, ou $R_1$ et $R_2$, lorsqu'ils sont pris ensemble avec les atomes de carbone auxquels ils sont liés, forment un composé cyclique phénylique;

Z représente CH ou N;

X représente —O—, —$CH_2Z_1$, —HC=CH—, —C≡C— ou —$CH_2Z_1CH_2$—;

Y représente —$CHCH_2$—, —C=CH—, —$CHCHR_5$ ou —$CHC(R_5)_2$— lorsque $R_3$ est monovalent ou —C—$CH_2$ lorsque $R_3$ représente O;

$R_3$ représente O, OH, $OR_4$, NH, $HNR_4$ ou $N(R_4)_2$ et M est un nombre entier allant de 0 à 5; dans lesquels $Z_1$ représente O, S ou $NR_4$; $R_4$ représente H, un alkyle, un pyranyle ou un aryle; et $R_5$ est H, un alkyle ou le fluor, à la condition que quand $R_3$ est OH, M est égal à 3 et la relation entre X et Y est du type méta ou para, $R_1$ ou $R_2$ est alors autre que de l'hydrogène.

2. Composés selon la revendication 1, de formule:

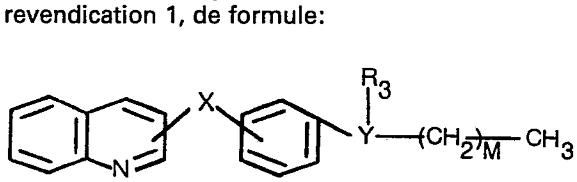

et leurs sels phamaceutiquement acceptables, formule dans laquelle X, Y, $R_3$ et M sont définis come dans la revendication 1.

3. Composés selon la revendication 1 ou 2, dans lesquels les groupes carboxyalkyle, carbalkoxyalkyle, alkylamino, aminoalkyle ou carbalkoxy contiennent jusqu'à 6 atomes de carbone.

4. Composés selon la revendication 1 ou 2, dans lesquels le groupe halogène dans l'halogénométhyle et dans le trihalogénométhyle et F, Cl, Br ou I.

5. Composés selon la revendication 1 ou 2, dans lesquels le groupe aryle en $R_4$ est le phényle ou le naphthyle.

6. Composés selon la revendication 1 ou 2 et leurs sels pharmaceutiquement acceptables, dans lesquels

$R_1$ et $R_2$ représentent, d'une manière indépendante, H, un carboxy, un carboxyalkyle, un carbalkoxy ou $R_1$ et $R_2$ forment un composé cyclique phénylique avec les atomes de carbone auxquels ils sont liés;

X représente $-CH{=}CH-$ ou $-CH_2Z_1$;

Y représente $-CHCH_2$ ou $-CHC(R_5)_2-$;

$R_3$ représente OH ou $HNR_4$ et

M est un nombre entier allant de 1 à 5.

7. Composés selon la revendication 1 ou 2, dans lesquels $R_4$ représente H, le méthyle, l'éthyle, le propyle ou le phényle.

8. Composés selon la revendication 1 ou 2 et leurs sels pharmaceutiquement acceptables, dans lesquels

$R_1$ et $R_2$ forment un composé cyclique phénylique avec les atomes de carbone auxquels ils sont liés;

X représente $CH_2Z_1$;

Y représente $CHCH_2$;

$R_3$ représente OH ou $HNR_4$ et

M est égal à 2 ou 3 et

$Z_1$ représente 0.

9. Méthyl-3-[[3-(1-hydroxyhexyl)phénoxy]-méthyl]-benzoate.

10. Méthyl-2-[[3-(1-hydroxyhexyl)phénoxy]-méthyl]-benzoate.

11. 2-[[3-(1-hydroxyhexyl)phénoxy]-méthyl]-pyridine.

12. 2-[[3-(1-hydroxyhexyl)phénoxy]-méthyl]-quinoline et ses sels pharmaceutiquement acceptables.

13. Composés de la formule générale I selon la revendication 1, dans lesquels

$R_1$ est $3\text{-}CO_2CH_3$, $R_2$ est l'hydrogène, Z est CH, X est $CH_2O$, Y est $3\text{-}CHCH_2$, $R_3$ est $OCOCH_3$ ou du tétrahydropyranoyle et M est égal à 3; ou

$R_1$ est $2\text{-}CO_2CH_3$, $R_2$ est l'hydrogène, Z est CH, X est $3\text{-}CHCH_2$, $R_3$ est OH et M est égal à 3; ou

$R_1$ et $R_2$ sont de l'hydrogène, Z est CH, X est $OCH_2$, Y est $3\text{-}CHCH_2$, $R_3$ est OH et M est égal à 3; ou

$R_1$ est $3\text{-}CO_2CH_3$, $R_2$ est l'hydrogène, Z est CH, X est $CH_2O$, Y est $3\text{-}CH{=}CH$, $R_3$ est $CH_3$ et M est égal à 3; ou $R_1$ est $3\text{-}CO_2CH_3$, $R_2$ est l'hydrogène, Z est CH, X est $CH_2O$, Y est $4{-}CH{=}CH$, $R_3$ est l'hydrogène et M est égal à 3.

14. Composition thérapeutique comprenant comme constituant actif un composé selon l'une quelconque des revendications 1 à 13.

15. Procédé de préparation d'un composé selon l'une quelconque des revendications 1 à 12, comprenant au moins l'une des phases suivantes:

(a) condensation d'un composé répondant aux formules II ou III:

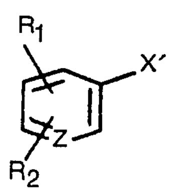

(II)

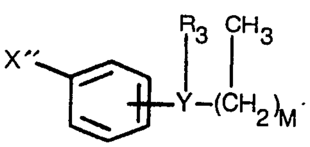

(III)

dans lesquelles X' et X'' représentent $-CH_2Hal$, Hal, OH, $CH_2OH$, SH ou $CH_2SH$;

(b) condensation d'un composé répondant à la formule II ou III ci-dessus, dans lesquelles X' et X'' représentent $-CH{=}CH_2$, $-C{\equiv}CH$ ou Hal;

(c) condensation d'un composé répondant à la formule II ou III ci-dessus, dans lesquelles X' et X'' représentent $-CHO$ ou $-NH_2$;

(d) condensation d'un composé répondant à la formule II ou III ci-dessus, dans lesquelles X' et X'' représentent COOH ou $Z_1H$;

(e) condensation d'un composé répondant à la formule II ou III ci-dessus, dans lesquelles X' et X'' représentent H ou un groupe d'acylation;

(f) condensation d'un composé répondant à la formule II ou III ci-dessus, dans lesquelles X' et X'' représentent $-NR_4H$ ou l'halogène;

(g) acylation ou alkylation d'un composé de formule IV

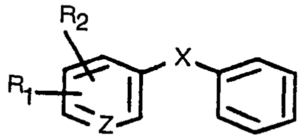

(IV)

pour introduire le substituant $—Y(R_3)-(CH_2)_MCH_3$; et éventuellement formation de sels, en particulier des sels pharmaceutiquement acceptables des produits formant des sels ainsi obtenus; et éventuellement introduction de substituants $R_1$ et $R_2$ au moyen de réactions de substitution connues.

**Revendications pour l'Etat contractant: AT**

1. Procédé de préparation de composés de formule générale I:

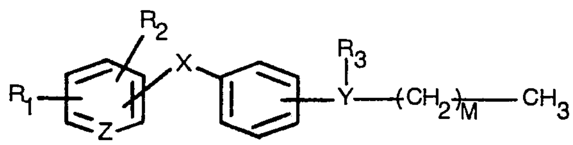

(I)

et de leurs sels pharmaceutiquement acceptables, formule dans laquelle

$R_1$ et $R_2$ représentent, d'une manière indépendante, un hydrogène, un alkyle, un carboxy, un carboxyalkyle, un carbalkoxyalkyle, un carbalkoxy, un formyle, un amino, un aminoalkyle, un alkylamino, un carboxamide, un halogène, un trihalométhyle, un hydroxy, ou $R_1$ et $R_2$, lorsqu'ils sont pris ensemble avec les atomes de carbone auxquels ils sont liés, forment un composé cyclique phénylique;

Z représente CH ou N;

X représente —O—, $—CH_2Z_1$, —HC=CH—, —C≡C— ou $—CH_2Z_1CH_2—$;

Y représente $—CHCH_2—$, —C=CH—, $—CHCHR_5$ ou $—CHC(R_5)_2—$ lorsque $R_3$ est monovalent ou $—C—CH_2$ lorsque $R_3$ représente O;

$R_3$ représente O, OH, $OR_4$, NH, $HNR_4$ ou $N(R_4)_2$ et M est un nombre entier allant de 0 à 5; dans lesquels $Z_1$ représente O, S ou $NR_4$; $R_4$ représente H, un alkyle, un pyranyle ou un aryle; et $R_5$ représente H, un alkyle ou le fluor, à la condition que quand $R_3$ est OH, M est égal à 3 et la relation entre X et Y est du type méta ou para, $R_1$ ou $R_2$ est alors autre que de l'hydrogène, ledit procédé comprenant au moins une des phases suivantes:

(a) condensation d'un composé de formule II:

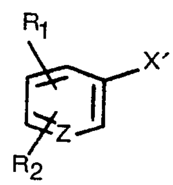

(II)

et III

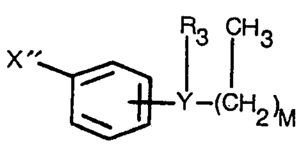

(III)

dans lesquelles X' et X'' représentent $—CH_2Hal$, Hal, OH, $CH_2OH$, SH ou $CH_2SH$;

(b) condensation d'un composé répondant à la formule II ou III ci-dessus, dans lesquelles X' et X'' représentent $—CH=CH_2$, —C≡CH ou Hal;

(c) condensation d'un composé répondant à la formule II ou III ci-dessus, dans lesquelles X' et X'' représentent —CHO ou $—NH_2$;

(d) condensation d'un composé répondant à la formule II ou III ci-dessus, dans lesquelles X' et X'' représentent COOH ou $Z_1H$;

(e) condensation d'un composé répondant à la formule II ou III ci-dessus, dans lesquelles X' et X'' représentent H ou un groupe d'acylation;

(f) condensation d'un composé répondant à la formule II ou III ci-dessus, dans lesquelles X' et X'' représentent $—NR_4H$ ou un l'halogène;

(g) acylation ou alkylation d'un composé de formule IV

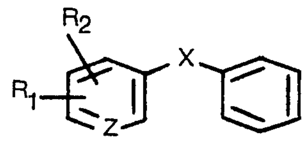

(IV)

pour introduire le substituant —$Y(R_3)$-$(CH_2)_MCH_3$; et éventuellement formation de sels, en particulier des sels pharmaceutiquement acceptables des produits formant des sels ainsi obtenus; et éventuellement introduction de substituants $R_1$ et $R_2$ au moyen de réactions de substitution connues.

2. Procédé selon la revendication 1, caractérisé en ce que le produit est un composé de formule:

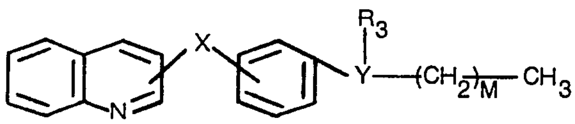

et ses sels pharmaceutiquement acceptables, formule dans laquelle X, Y, $R_3$ et M sont définis comme dans la revendication 1.

3. Procédé selon la revendication 1 ou 2, caractérisé en ce que les groupes carboxyalkyle, carbalkoxyalkyle, alkylamino, aminoalkyle ou carbalkoxy contiennent jusqu'à 6 atomes de carbone.

4. Procédé selon la revendication 1 ou 2, caractérisé en ce que le groupe halogène dans l'halogénométhyle et dans le trihalogénométhyle est F, Cl, Br ou I.

5. Procédé selon la revendication 1 ou 2, caractérisé en ce que le groupe aryle en $R_4$ est le phényle ou le naphthyle.

6. Procédé selon la revendication 1 ou 2, caractérisé en ce que

$R_1$ et $R_2$ représentent, d'une manière indépendante, H, un carboxy, un carboxyalkyle, un carbalkoxy ou $R_1$ et $R_2$ forment un composé cyclique phénylique avec les atomes de carbone auxquels ils sont liés;

X représente —CH=CH— ou —$CH_2Z_1$;

Y représente —$CHCH_2$ ou —$CHC(R_5)_2$—;

$R_3$ représente OH ou $HNR_4$ et

M est un nombre entier allant de 1 à 5.

7. Procédé selon la revendication 1 ou 2, caractérisé en ce que $R_4$ représente H, le méthyle, l'éthyle, le propyle ou le phényle.

8. Procédé selon la revendication 1 ou 2, caractérisé en ce que $R_1$ et $R_2$ représentent, d'une manière indépendante, H, ou $R_1$ et $R_2$ forment un composé cyclique phenylique avec les atomes de carbone auxquels ils sont liés;

X représente $CH_2Z_1$;

Y représente $CHCH_2$;

$R_3$ représente OH ou $HNR_4$ et

M est un nombre entier égal à 2 ou 3 et

$Z_1$ représente 0.

9. Procédé selon la revendication 1, caractérisé en ce que le produit est le méthyl-3-[[3-(1-hydroxyhexyl)phénoxy]-méthyl]-benzoate.

10. Procédé selon la revendication 1, caractérisé en ce que le produit est le méthyl-2-[[3-(1-hydroxyhexyl)phénoxy]-méthyl]-benzoate.

11. Procédé selon la revendication 1, caractérisé en ce que le produit est la 2-[[3-(1-hydroxyhexyl)phénoxy]-méthyl]-pyridine.

12. Procédé selon la revendication 1, caractérisé en ce que le produit est la 2-[[3-(1-hydroxyhexyl)phénoxy]-méthyl]-quinoline et ses sels pharmaceutiquement acceptables.

13. Procédé de préparation d'une composition thérapeutique comprenant comme agent actif un composé selon l'une quelconque des revendications 1 à 12 et des supports et/ou diluants pharmaceutiquement acceptables.